(19) Europäisches Patentamt
European Patent Office
Office européen des brevets

(11) Veröffentlichungsnummer: **0 096 204**
**B1**

# (12) EUROPÄISCHE PATENTSCHRIFT

(45) Veröffentlichungstag der Patentschrift: **17.09.86**

(51) Int. Cl.⁴: **C 07 D 233/72,** C 08 G 18/38

(21) Anmeldenummer: **83103985.4**

(22) Anmeldetag: **23.04.83**

## (54) Verfahren zur Herstellung von blockierte und gegebenenfalls freie Hydroxylgruppen aufweisenden Verbindungen, sowie ihre Verwendung zur Herstellung von Polyurethankunststoffen.

(30) Priorität: **08.05.82 DE 3217387**

(43) Veröffentlichungstag der Anmeldung: **21.12.83 Patentblatt 83/51**

(45) Bekanntmachung des Hinweises auf die Patenterteilung: **17.09.86 Patentblatt 86/38**

(84) Benannte Vertragsstaaten: **AT BE DE FR GB IT NL SE**

(56) Entgegenhaltungen: **DE - A - 1 936 157**

(73) Patentinhaber: **BAYER AG, Konzernverwaltung RP Patentabteilung, D-5090 Leverkusen 1 Bayerwerk (DE)**

(72) Erfinder: **Buschhaus, Hans-Ulrich, Dr., Morgengraben 2, D-5000 Köln 80 (DE)**
Erfinder: **Findeisen, Kurt, Dr., In der Follmühle 10, D-5068 Odenthal (DE)**
Erfinder: **Traenckner, Hans-Joachim, Dr., Wedelstrasse 46, D-4150 Krefeld (DE)**
Erfinder: **Beer, Wolfgang, Dr., Heyenfeldweg 132, D-4150 Krefeld (DE)**

EP 0 096 204 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von blockierte alkoholische Hydroxylgruppen und gegebenenfalls freie Hydroxylgruppen aufweisenden Verbindungen und die Verwendung von insgesamt mindestens zwei derartige Gruppen aufweisenden Verbindungen zur Herstellung von Polyurethankunststoffen.

Es ist allgemein bekannt, bei Raumtemperatur lagerfähige, unter der Einwirkung von Hitze in hochmolekulare Polyurethane überführbare Reaktivsysteme dadurch herzustellen, dass man organische Polyhydroxylverbindungen mit vorzugsweise alkoholisch gebundenen Hydroxylgruppen mit blockierten Polyisocyanaten vermischt (vgl. z.B. Houben-Weyl, Methoden der organischen Chemie, Band XIV/2, 4. Auflage, Georg Thieme Verlag, Stuttgart 1963, Seiten 61–70). Nachteilig hierbei ist aber, dass bei der thermischen Aushärtung derartiger Systeme flüchtige, oft auch brennbare und physiologisch bedenkliche Substanzen, d.h. die zur Blockierung der Isocyanatgruppen eingesetzten Blockierungsmittel freigesetzt werden. Die Verwendung derartiger Systeme in Beschichtungsmitteln ist ausserdem mit dem Nachteil behaftet, dass eine chemische Antrocknung der Beschichtung, z.B. durch Reaktion von freien Isocyanatgruppen mit Luftfeuchtigkeit nicht stattfinden kann, da die Systeme unterhalb der Aushärttemperatur auch gegenüber Luftfeuchtigkeit weitgehend inert sind.

Es war daher die der Erfindung zugrundeliegende Aufgabe, neue Systeme zur Verfügung zu stellen, die nicht mit den Nachteilen der klassischen Systeme auf Basis von blockierten Polyisocyanaten und freien Polyhydroxylverbindungen behaftet sind, d.h. die bei praktisch beliebig langer Verarbeitbarkeit bei Raumtemperatur ohne Abspaltung von leicht flüchtigen Blockierungsmitteln thermisch in hochmolekulare Polyurethane überführbar sind, und die aufgrund des Vorliegens von freien Isocyanatgruppen bereits vor der thermischen Vernetzung unter dem Einfluss von Luftfeuchtigkeit «chemisch antrocknen».

Überraschenderweise wurde nun gefunden, dass diese Aufgabe dadurch gelöst werden kann, dass man die in der Polyurethanchemie üblichen Polyisocyanate mit freien Isocyanatgruppen mit bestimmten, nach dem nachstehend näher beschriebenen Verfahren erhaltenen Verbindungen mit blockierten alkoholischen Hydroxylgruppen kombiniert.

Die nach dem nachstehend näher beschriebenen erfindungsgemässen Verfahren erhaltenen Verbindungen mit blockierten und gegebenenfalls zusätzlich freien alkoholischen Hydroxylgruppen eignen sich nicht nur für das genannte Einsatzgebiet, sondern stellen – dies gilt insbesondere für erfindungsgemässe Verfahrensprodukte auf Basis von mehrwertigen Alkoholen und mindestens difunktionellen N,N'-disubstituierten 5-Acylimino-imidazolidindionen – hochmolekulare, oft kautschukähnliche Polymere dar, die durch einfaches Erhitzen in ihre monomeren Bestandteile zerlegt werden können.

Gegenstand der Erfindung ist ein Verfahren zur Herstellung von reversibel blockierte und gegebenenfalls freie alkoholische Hydroxylgruppen aufweisenden Verbindungen, das dadurch gekennzeichnet ist, dass man gegebenenfalls Ether- oder Esterbrücken aufweisende 2- bis 6-wertige aliphatische Alkohole eines Molekulargewichts von 62–5000 oder Gemische derartiger Alkohole bei 0 bis 140°C mit N,N'-disubstituierten 5-Acylimino-imidazolidindionen der allgemeinen Formel

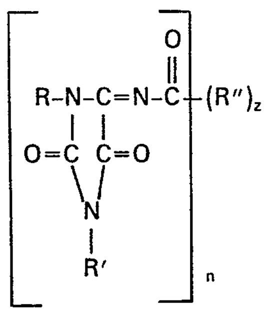

in der

R und R' gleich oder verschieden sind und einen gegebenenfalls durch Halogen, $C_1$–$C_4$-Alkyl-, Methoxy-, Nitro-, $C_1$–$C_4$-Carbalkoxy- oder Nitrilgruppen substituierten aliphatischen Kohlenwasserstoffrest mit 1–20 Kohlenstoffatomen, cycloaliphatischen Kohlenwasserstoffrest mit 3–15 Kohlenstoffatomen, aromatischen Kohlenwasserstoffrest mit 6–15 Kohlenstoffatomen oder araliphatischen Kohlenwasserstoffrest mit 7–15 Kohlenstoffatomen darstellen,

R" für einen gegebenenfalls inerte Substituenten aufweisenden, n-wertigen aliphatischen Kohlenwasserstoffrest mit 2–20, vorzugsweise 2–6 Kohlenstoffatomen, cycloaliphatischen Kohlenwasserstoffrest mit 3–15, vorzugsweise 6 Kohlenstoffatomen, aromatischen Kohlenwasserstoffrest mit 6–15, vorzugsweise 6–10 Kohlenstoffatomen, araliphatischen Kohlenwasserstoffrest mit 7–15, vorzugsweise 7–8 Kohlenstoffatomen, einen gegebenenfalls inerte Substituenten aufweisenden Alkoxyrest mit 1–4 Kohlenstoffatomen, einen Phenoxyrest oder einen Rest steht, wie er durch Entfernung der Chlorcarbonyl-Gruppen von einem n-funktionellen organischen Carbamidsäurechlorid mit insgesamt 2–10 Kohlenstoffatomen erhalten wird,

n für eine ganze Zahl von 1–3 steht und

z 0 oder 1 bedeutet, zur Reaktion bringt.

Gegenstand der Erfindung ist auch die Verwendung der nach diesem Verfahren erhaltenen, insgesamt mindestens 2 blockierte und freie Hydroxylgruppen aufweisenden Verbindungen zur Herstellung von Polyurethankunststoffen.

Beim erfindungsgemässen Verfahren werden beliebige, gegebenenfalls Ether- oder Esterbrükken aufweisende, 2- bis 6-wertige, insbesondere 2- bis 3-wertige aliphatische Alkohole des aus dem Hydroxylgehalt und der Funktionalität berechenbaren bzw., im Falle von verzweigten Polyesterpolyolen, osmometrisch bestimmbaren

(mittleren) Molekulargewichts von 62–5000, vorzugsweise 62–3000, eingesetzt. Dies bedeutet, dass es sich bei den einzusetzenden, alkoholische Hydroxylgruppen aufweisenden Verbindungen um die aus der Polyurethanchemie bekannten Polyhydroxylverbindungen handelt. Die beim erfindungsgemässen Verfahren einzusetzenden, Hydroxylgruppen aufweisenden Verbindungen besitzen vorzugsweise ausschliesslich primäre und/oder sekundäre Hydroxylgruppen.

Typische Beispiele für erfindungsgemäss geeignete Verbindungen mit alkoholischen Hydroxylgruppen sind

1. Primäre oder sekundäre Dihydroxyverbindungen mit alkoholisch gebundenen Hydroxylgruppen des Molekulargewichtsbereichs 62–300 wie Glykol, 2,2'-Dihydroxy-diethylether, 1,2-Bis-(2-hydroxyethoxyethan, Tetraethylenglykol, Bis-(2-hydroxyethyl)-sulfid, 1,2-Propandiol, Dipropylenglykol, Tripropylenglykol, 3-Chlor-1,2-propandiol, 1,3-Propandiol, 1,2-Butandiol, 1,3-Butandiol, 1,4-Butandiol, 2,3-Butandiol, 1,5-Pentandiol, 2,2-Dimethyl-1,3-propandiol, 1,6-Hexandiol, 2,5-Hexandiol, 2,2-Diethyl-1,3-propandiol, 1,12-Octandiol, 2-Buten-1,4-diol, 2-Butin-1,4-diol, 1,2-Cyclohexandiol oder 1,4-Cyclohexandiol.

2. Primäre und/oder sekundäre drei- oder mehrwertige Alkohole des Molekulargewichtsbereichs 92–350 wie Glycerin, 2-Hydroxymethyl-2-methyl, 1,3-propandiol, 1,2,6-Hexantriol, 2-Ethyl-2-hydroxymethyl-1,3-propandiol, 2,2-Bis-hydroxymethyl-1,3-propandiol, Sorbit, Mannit, Rohrzucker, Glucose und Fructose.

3. Polyester- oder Polyetherpolyole des Molekulargewichtsbereichs 300–5000, vorzugsweise 1000–3000, welche 2–6, vorzugsweise 2–3 alkoholische Hydroxylgruppen aufweisen, und wie sie aus der Polyurethanchemie an sich bekannt sind (vgl. z.B. US-PS 4 218 543, Kolonne 7, Zeile 29 – Kolonne 9, Zeile 25).

Reaktionspartner für diese Verbindungen mit alkoholischen Hydroxylgruppen sind N,N'-disubstituierte 5-Acylimino-imidazolidindione der o.g. allgemeinen Formel. Bevorzugte derartige Verbindungen sind solche der genannten allgemeinen Formel, für welche R und R' für gleiche oder verschiedene Reste stehen und jeweils Alkylgruppen mit 1–4 Kohlenstoffatomen bedeuten,

R'' für einen n-wertigen, unsubstituierten, gesättigten aliphatischen Kohlenwasserstoffrest mit 1–6 Kohlenstoffatomen oder einen n-wertigen, unsubstituierten aromatischen Kohlenwasserstoffrest mit 6 Kohlenstoffatomen steht,
n für 1 oder 2 steht und
z für 1 steht.

Beispielhaft seien als, für das erfindungsgemässe Verfahren geeignete, N,N'-disubstituierte 5-Acyliminoimidazolidindione genannt:

N,N'-Dimethyl-5-acetylimino-imidazolidindion,
N,N'-Dibutyl-5-acetylimino-imidazolidindion,
N,N'-Distearyl-5-acetylimino-imidazolidindion,
N,N'-Dimethyl-5-benzoylimino-imidazolidindion,
N,N'-Dibutyl-5-benzoylimino-imidazolidindion,
das 2:1-Umsetzungsprodukt von N,N'-Dibutyl-5-trimethylsilylimino-imidazolidindion mit Bernsteinsäuredichlorid, das 2:1-Umsetzungsprodukt von N,N'-Dibutyl-5-trimethylsilyl-imino-imidazolidindion mit Adipinsäuredichlorid, das 2:1-Umsetzungsprodukt von N,N'-Dimethyl-5-trimethylsilylimino-imidazolidindion mit Terephthalsäuredichlorid oder das 3:1-Umsetzungsprodukt von N,N'-Dimethyl-5-trimethylsilyl-imino-imidazolidindion mit Trimesinsäuretrichlorid.

Die N,N'-disubstituierten 5-Acylimino-imidazolidindione sind im Prinzip beispielsweise aus der DE-OS 1 936 157 bekannt. Sie können entweder aus 5-Imino-imidazolidindionen durch Umsetzung mit Ketenen oder durch Umsetzung von 5-Trimethylsilylimino-imidazolidindionen mit Säurehalogeniden hergestellt werden (vgl. Deutsche Patentanmeldung P 3 211 911.9).

Bei der erfindungsgemässen Umsetzung erfolgt eine Anlagerung der alkoholischen Hydroxylgruppe an die 5-Acylimino-Gruppe wie nachstehend am Beispiel der Reaktion zwischen N,N'-Dimethyl-5-acetylimino-imidazolidindion und Ethanol erläutert:

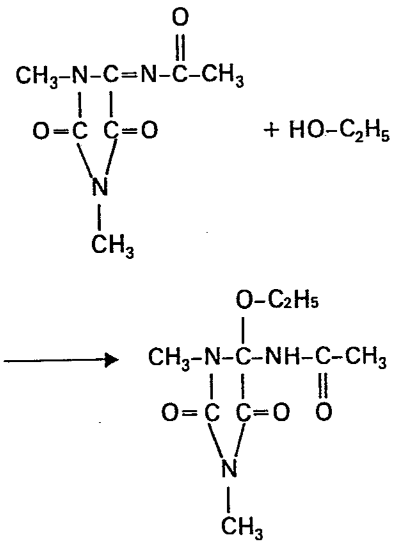

Diese Additionsreaktion ist reversibel, so dass die Additionsverbindungen beim Erhitzen auf oberhalb 140°C liegende Temperaturen den angelagerten Alkohol abspalten.

Das erfindungsgemässe Verfahren kann in Gegenwart von Lösungsmitteln oder lösungsmittelfrei durchgeführt werden. Gegebenenfalls einzusetzende Lösungsmittel sind z.B. halogenierte Kohlenwasserstoffe wie Methylenchlorid, Chloroform, Trichlorethan, Chlorbenzol, 1,2-Dichlorbenzol, Toluol, Xylol, Hexan, Petrolether, Waschbenzin, Benzin und Benzingemische, wie sie in der Lackindustrie verwendet werden, Ethylglykolacetat, Glykolmonomethyletheracetat und/oder Gemische der genannten Lösungsmittel. Bei der Verwendung von leicht destillierbaren einwertigen Alkoholen beim erfindungsgemässen Verfahren können diese auch in hohem Überschuss eingesetzt werden, wobei die überschüssigen Mengen die Funktion eines Lösungsmittels übernehmen.

Die erfindungsgemässe Umsetzung kann im Temperaturbereich von 0-140°C, bevorzugt 20–130°C und besonders bevorzugt im Bereich von 80–110°C durchgeführt werden.

Bei der Durchführung des erfindungsgemässen Verfahrens kommen die Reaktionspartner im allgemeinen in einem Äquivalentverhältnis von Hydroxylgruppen zu Imidazoilidindion-Struktureinheiten von 0,9:1 bis 2:1, vorzugsweise 0,9:1 bis 1,5:1, insbesondere 1:1, entsprechenden Mengen zum Einsatz.

Die Wahl der Art und Mengenverhältnisse der beim erfindungsgemässen Verfahren einzusetzenden Ausgangsmaterialien richtet sich in erster Linie nach dem beabsichtigten Verwendungszweck für die erfindungsgemässen Verfahrensprodukte.

Verfahrensprodukte, die der erfindungsgemässen Verwendung zugeführt werden sollen, d.h. die mit organischen Polyisocyanaten zu Hitze-vernetzbaren Systemen kombiniert werden sollen, werden unter Verwendung von monofunktionellen erfindungswesentlichen Blockierungsmitteln (n = 1) hergestellt. Hierbei ist es nicht erforderlich, alle freien Hydroxylgruppen zu blockieren, so dass auch unter Verwendung eines Überschusses an Hydroxylverbindungn innerhalb der o.g. Grenzen gearbeitet werden kann. Auf diese Weise erhaltene, lediglich teilblockierte Polyhydroxylverbindungen reagieren bei unterhalb 140°C liegenden Temperaturen, insbesondere bei Raumtemperatur, selbstverständlich nur teilweise mit dem Polyisocyanat ab, so dass auch nach dieser Teilreaktion noch verarbeitbare Systeme vorliegen. Eine derartige unvollständige Vorreaktion (Addition eines Teils der Isocyanatgruppen an die freien Hydroxylgruppen) kann in besonderen Fällen erwünscht sein, beispielsweise wenn hochviskose Systeme, beispielsweise Dichtungsmassen, hergestellt werden sollen, die erst bei hoher Temperatur in den vernetzten Zustand übergehen.

Bei der Verwendung von mehrwertigen Alkoholen in Kombination mit mindestens difunktionellen erfindungswesentlichen Blockierungsmitteln (n = 2 oder 3) entstehen unter Einhaltung eines Äquivalentverhältnisses von 0,9:1 bis 1,1:1, insbesondere von ca. 1:1, neuartige Hochmolekulare, oft kautschukartige Polymere, die beim Erhitzen auf über 140°C in ihre monomeren Bestandteile zerfallen. Die erfindungsgemässen Verfahrensprodukte können im allgemeinen, gegebenenfalls nach destillativer Entfernung des mitverwendeten Lösungsmittels, ohne weitere Reinigungsoperation ihrem jeweiligen Verwendungszweck, beispielsweise der erfindungsgemässen Verwendung, zugeführt werden.

Bei der erfindungsgemässen Verwendung der insgesamt mindestens 2 blockierte und freie Hydroxylgruppen aufweisenden erfindungsgemässen Verfahrensprodukte werden diese z.B. mit den aus der Polyurethanchemie an sich bekannten Polyisocyanaten unter Einhaltung eines Äquivalentverhältnisses von Isocyanatgruppen zu freien und blockierten Hydroxylgruppen von 2:1 bis 0,5:1, vorzugsweise 1,1:1 bis 0,9:1, vermischt. Diesen Mischungen können zusätzlich die aus der Polyurethanchemie an sich bekannten Hilfs- und Zusatzmittel, wie z.B. Lösungsmittel, Pigmente, Füllstoffe, Verlaufshilfsmittel, u.dgl. zugesetzt werden.

Für die erfindungsgemässe Verwendung besonders gut geeignete organische Polyisocyanate sind insbesondere 2,4- und/oder 2,6-Diisocyanatotoluol, 4,4'-Diisocyanatodiphenylmethan und dessen technische Gemische mit 2,4'-Diisocyanatodiphenylmethan und/oder höheren Homologen, Hexamethylendiisocyanat, Biuretpolyisocyanat auf Basis von Hexamethylendiisocyanat, Isophorondiisocyanat oder 4,4'-Diisocyanato-cyclohexyl-methan. NCO-Prepolymere auf Basis derartiger Polyisocyanate und den oben unter 2. bis 4. beispielhaft offenbarten Hydroxylverbindungen sind ebenfalls geeignete Polyisocyanate.

Die erfindungsgemässe Verwendung der erfindungsgemässen Verfahrensprodukte zur Herstellung von Polyurethankunststoffen eignet sich insbesondere zur Herstellung von Hitze-vernetzbarben Einbrennlacken. Derartige Einbrennlacke sind, abgesehen von der im allgemeinen nicht störenden Vorreaktion zwischen einem Teil der Isocyanatgruppen und gegebenenfalls vorliegenden freien Hydroxylgruppen, bei unterhalb 140°C, insbesondere bei Raumtemperatur, praktisch unbegrenzt lagerfähig und können zu einem beliebigen Zeitpunkt nach ihrer Herstellung verarbeitet werden. Mit derartigen Systemen auf hitzeresistente Substrate nach beliebigen Methoden der Lacktechnologie aufgetragene Überzüge härten nach ca. 15- bis 60-minütigem Erhitzen auf oberhalb 140°C, insbesondere 145–180°C zu hochwertigen Lacküberzügen aus. Die hierbei freigesetzten erfindungswesentlichen Blockierungsmittel stellen schwerflüchtige Verbindungen dar, die auch unter den genannten Einbrennbedingungen in der Lackschicht verbleiben, ohne deren lacktechnischen Eigenschaften negativ zu beeinträchtigen.

Die nachfolgenden Beispiele dienen zur weiteren Erläuterung der Erfindung. Alle Prozentangaben beziehen sich, soweit nicht anders vermerkt, auf Gewichtsprozente.

Beispiel 1

100 g (0,294 Hydroxyläquivalente) eines handelsüblichen Polyesters des osmometrisch bestimmten Molekulargewichts 1580 aus Phthalsäureanhydrid, Maleinsäureanhydrid (Molverhältnis = 2:1) und Trimethylolpropan, 65%ig in Ethylglykolacetat, OH-Zahl 165, werden bei 100°C mit 78,5 g N,N'-Dibutyl-5-acetyliminoimidazolidindion (0,294 Mol) 5 Stunden gerührt. Nach Abkühlen auf Raumtemperatur erhält man eine klare, viskose Lösung. OH-Zahl: 11.

Beispiel 2

20 g (entsprechend 0,033 OH-Äquivalenten, bezogen auf blockierte und freie Hydroxylgruppen) des Umsetzungsproduktes aus Beispiel 1 werden mit 6,2 g eines niederviskosen biuretisierten He-

xamethylendiisocyanats mit einem NCO-Gehalt von 23,4% (entsprechend 0,035 NCO-Äquivalenten) gemischt, mit 20 g einer Mischung von Ethylglykolacetat/Xylol 1:1 verdünnt und auf eine Platte aufgestrichen. Nach dem Aushärten bei 150°C während 30 Minuten erhält man einen klaren, ofentrockenen und elastischen Film, der von Aceton oder Xylol nicht angelöst wird.

Die Mischung ist nach Lagerung von 2 Monaten bei 25°C noch gut verarbeitbar.

Vergleichsbeispiel

20 g des in Beispiel 1 verwendeten Polyesters (entsprechend 0,059 OH-Äquivalenten) werden mit 10,6 g des niederviskosen biuretisierten Hexamethylendiisocyanats (entsprechend 0,062 NCO-Äquivalenten) gemischt und mit 20 g Ethylglykolacetat/Xylol 1:1 verdünnt. Die Mischung ist nach einem Tag bei 25°C gummiartig erstarrt.

Beispiel 3

500 g eines auf 1,2-Propandiol gestarteten Polyethers mit 95% Propylen- und 5% Ethylenoxid-Einheiten, einer mittleren Hydroxylzahl von 56 und einem Gehalt an primären Hydroxylgruppen von ca. 45%, sowie einer Viskosität von 300 cP/25°C werden mit 133,5 g (0,5 Mol) 5-Acetylimino-N,N′-dibutylimidazolidindion bei 100°C gerührt. Nach 10 h ist die Mischung frei von Hydroxylgruppen.

Beispiel 4

20 g des Umsetzungsprodukts aus Beispiel 3, 20 g Ethylglykolacetat und 3,03 g eines Biuretpolyisocyanats auf Basis von Hexamethylendiisocyanat mit einem NCO-Gehalt von 22,98% werden gemischt und auf eine entfettete Glasplatte aufgestrichen. Die Beschichtung wird 30 Min. bei 160°C eingebrannt. Man erhält einen weichen, sehr elastischen Überzug.

Beispiel 5

140 g eines Polyesters aus Adipinsäure, Phthalsäureanhydrid, Maleinsäureanhydrid, Propylenglykol-1,2 und Trimethylolpropan mit einer Hydroxylzahl von 290 werden in 60 g Ethylglykolacetat gelöst und mit 166,6 g 5-Acetylimino-N,N′-dibutyl-imidazolidindion bei 100°C gerührt. Nach 8 h Rühren ist die Mischung frei von Hydroxylgruppen.

Beispiel 6

20 g des Produkts aus Beispiel 5, 20 g Ethylglykolacetat und 6,53 g des Biuretpolyisocyanats auf Basis von Hexamethylendiisocyanat mit einem NCO-Gehalt von 22,98% werden gemischt und die Mischung auf eine entfettete Glasplatte aufgestrichen. Die Beschichtung wird 30 Min. bei 160°C eingebrannt. Man erhält eine klare, farblose und lösungsmittelbeständige Beschichtung.

Beispiel 7

80,1 g (0,3 Mol) 5-Acetylimino-N,N′-dibutylimidazolidindion, 100 ml Toluol und 9,2 g (0,1 Mol) Glycerin werden während 16 Stunden bei 120°C gerührt und das Toluol bei 20 mbar abdestilliert. Es verbleibt ein hellgelber, hochviskoser und Hydroxylgruppen-freier Rückstand.

Beispiel 8

45 g (0,5 Mol) 1,4-Dihydroxybutan, 267 g (1 Mol) 5-Acetylimino-N,N′-dibutylimidazolidindion und 200 ml Toluol werden 8 h bei 120°C gerührt, das Toluol abdestilliert und der Rückstand im Vakuum bei 70°C getrocknet. Es resultiert ein weisser, Hydroxylgruppen-freier Rückstand mit einem Schmelzpunkt von 137°C.

Beispiel 9

7,06 g des Produkts aus Beispiel 8 werden in 35 g Dichlormethan gelöst. In einem getrennten Ansatz wird durch Umsetzung des Biuretpolyisocyanats gemäss Beispiel 6 mit einer unterschüssigen Menge eines Polyesters auf Basis Phthalsäureanhydrid, Maleinsäureanhydrid und Trimethylolpropan (Hydroxylzahl: 260) ein NCO-Prepolymer mit einem NCO-Gehalt von 9,5% hergestellt. Dieses NCO-Prepolymer wird 60%ig in einer Mischung aus Ethylenglykolacetat/Xylol (4:1) gelöst. Beide Lösungen werden schliesslich zu einem Beschichtungsmittel vereinigt (Äquivalentverhältnis NCO-Gruppen/blockierte Hydroxylgruppen = 1:1).

Die Mischung wird auf eine entfettete Glasplatte gestrichen und während 30 Minuten bei 150°C eingebrannt. Man erhält einen klaren, elastischen und acetonfesten Überzug.

## Patentansprüche

1. Verfahren zur Herstellung von reversibel blockierte und gegebenenfalls freie alkoholische Hydroxylgruppen aufweisenden Verbindungen, dadurch gekennzeichnet, dass man gegebenenfalls Ether- oder Esterbrücken aufweisende 2- bis 6-wertige aliphatische Alkohole eines Molekulargewichts von 62–5000 oder Gemische derartiger Alkohole bei 0 bis 140°C mit N,N′-disubstituierten 5-Acylimino-imidazolidindionen der allgemeinen Formel

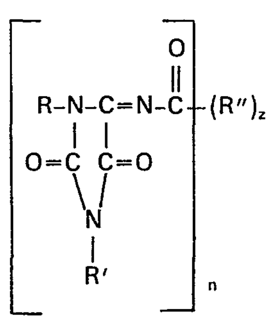

in der

R und R′ gleich oder verschieden sind und einen gegebenenfalls durch Halogen, $C_1$–$C_4$-Alkyl-,

Methoxy-, Nitro-, $C_1$–$C_4$-Carbalkoxy- oder Nitrilgruppen substituierten aliphatischen Kohlenwasserstoffrest mit 1–20 Kohlenstoffatomen, cycloaliphatischen Kohlenwasserstoffrest mit 3–15 Kohlenstoffatomen, aromatischen Kohlenwasserstoffrest mit 6–15 Kohlenstoffatomen oder araliphatischen Kohlenwasserstoffrest mit 7–15 Kohlenstoffatomen darstellen,

R″ für einen gegebenenfalls inerte Substituenten aufweisenden, n-wertigen aliphatischen Kohlenwasserstoffrest mit 2–20, vorzugsweise 2–6 Kohlenstoffatomen, cycloaliphatischen Kohlenwasserstoffrest mit 3–15, vorzugsweise 6 Kohlenstoffatomen, aromatischen Kohlenwasserstoffrest mit 6–15, vorzugsweise 6–10 Kohlenstoffatomen, araliphatischen Kohlenwasserstoffrest mit 7–15, vorzugsweise 7–8 Kohlenstoffatomen, einen gegebenenfalls inerte Substituenten aufweisenden Alkoxyrest mit 1–4 Kohlenstoffatomen, einen Phenoxyrest oder einen Rest steht, wie er durch Entfernung der Chlorcarbonyl-Gruppen von einem n-funktionellen organischen Carbamidsäurechlorid mit insgesamt 2–10 Kohlenstoffatomen erhalten wird,

n für eine ganze Zahl von 1–3 steht und

z 0 oder 1 bedeutet, zur Reaktion bringt.

2. Verfahren gemäss Anspruch 1, dadurch gekennzeichnet, dass man als N,N′-disubstituierte 5-Acylimino-imidazolidindione solche der in Anspruch 1 genannten allgemeinen Formel verwendet, für welche R und R′ für gleiche oder verschiedene Reste stehen und jeweils einen aliphatischen Kohlenwasserstoffrest mit 1–4 Kohlenstoffatomen bedeuten,

R″ für einen n-wertigen, unsubstituierten, gesättigten aliphatischen Kohlenwasserstoffrest mit 1–6 Kohlenstoffatomen oder für einen n-wertigen, unsubstituierten aromatischen Kohlenwasserstoffrest mit 6 Kohlenstoffatomen steht,

n für 1 oder 2 steht und

z für 1 steht.

3. Verfahren gemäss Anspruch 1 bis 2, dadurch gekennzeichnet, dass man die Reaktionspartner in einem Äquivalentverhältnis von alkoholischen Hydroxylgruppen zu Imidazolidindion-Struktureinheiten von 0,9:1 bis 1,5:1 entsprechenden Mengen einsetzt.

4. Verwendung der gemäss Anspruch 1 bis 3 erhaltenen, insgesamt mindestens 2 blockierte und freie Hydroxylgruppen aufweisenden Verbindungen als Ausgangsmaterial zur Herstellung von Polyurethankunststoffen.

## Claims

1. Process for the production of compounds containing reversibly blocked and, optionally, free alcoholic hydroxyl groups, characterised in that 2- to 6-hydric aliphatic alcohols which have a molecular weight of 62-5000 and optionally contain ether or ester bridges or mixtures of such alcohols are reacted at 0 to 140°C with N,N′-disubstituted 5-acylimino-imidazolidine diones of the general formula

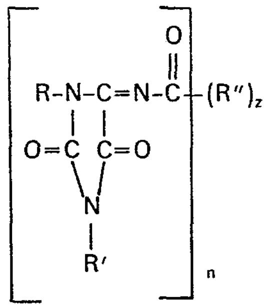

in which

R and R′ are identical or different and represent an optionally halogen-, $C_1$–$C_4$-alkyl-, methoxy-, nitro-, $C_1$–$C_4$-carbalkoxy- or nitrile-group-substituted aliphatic hydrocarbon radical with 1–20 carbon atoms, cycloaliphatic hydrocarbon radical with 3–15 carbon atoms, aromatic hydrocarbon radical with 6–15 carbon atoms or araliphatic hydrocarbon radical with 7–15 carbon atoms,

R″ represents a n-valent aliphatic hydrocarbon radical with 2–20, preferably 2–6 carbon atoms, cycloaliphatic hydrocarbon radical with 3–15, preferably 6 carbon atoms, aromatic hydrocarbon radical with 6–15, preferably 6–10 carbon atoms or araliphatic hydrocarbon radical with 7–15, preferably 7–8 carbon atoms, which radicals optionally contain inert substituents, an alkoxy radical with 1–4 carbon atoms which optionally contains inert substituents, a phenoxy radical or a radical of the type obtained by the removal of the chlorocarbonyl groups from a n-functional organic carbamic acid chloride with a total of 2–10 carbon atoms,

n represents an integer from 1–3 and

z denotes 0 or 1.

2. Process according to Claim 1, characterised in that the N,N′-disubstituted 5-acylimino-imidazolidine diones used are those of the general formula mentioned in Claim 1, in which R and R′ represent identical or different radicals and each denote an aliphatic hydrocarbon radical with 1–4 carbon atoms,

R″ represents a n-valent, unsubstituted, saturated aliphatic hydrocarbon radical with 1–6 carbon atoms or a n-valent, unsubstituted aromatic hydrocarbon radical with 6 carbon atoms,

n represents 1 or 2 and

z represents 1.

3. Process according to Claim 1 to 2, characterised in that the reactants are used in quantities corresponding to an equivalent ratio of alcoholic hydroxyl groups to imidazolidine dione structural units of 0.9:1 to 1.5:1.

4. Use of the compounds obtained according to Claim 1 to 3 and containing a total of at least 2 blocked and free hydroxyl groups as starting material for the production of polyurethane plastics.

## Revendications

1. Procédé de préparation de composés comportant des groupes hydroxy bloqués de manière réversible et éventuellement des groupes hydroxy alcooliques libres, caractérisé en ce qu'on fait réagir des alcools aliphatiques bivalents à hexavalents d'un poids moléculaire de 62–5.000 comportant éventuellement des ponts éther ou ester ou des mélanges de tels alcools, à 0–140°C, avec des 5-acylimino-imidazolidine-diones N,N'-disubstituées de formule générale:

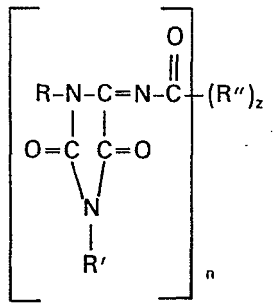

dans laquelle
R et R' sont identiques ou différents et représentent un radical d'hydrocarbure araliphatique contenant 7 à 15 atomes de carbone, un radical d'hydrocarbure aromatique contenant 6 à 15 atomes de carbone, un radical d'hydrocarbure cycloaliphatique contenant 3 à 15 atomes de carbone ou un radical d'hydrocarbure aliphatique contenant 1–20 atomes de carbone éventuellement substitué par un atome d'halogène, par un groupe alkyle en $C_1$–$C_4$, par un groupe méthoxy, par un groupe nitro, par un groupe carbalcoxy en $C_1$–$C_4$ ou par un groupe nitrile,
R″ représente un radical d'hydrocarbure araliphatique contenant 7–15, de préférence, 7–8 atomes de carbone, un radical d'hydrocarbure aromatique contenant 6–15, de préférence, 6–10 atomes de carbone, un radical d'hydrocarbure cycloaliphatique contenant 3–15, de préférence, 6 atomes de carbone, un radical d'hydrocarbure aliphatique n-valent contenant 2–20, de préférence, 2–6 atomes de carbone et comportant éventuellement des substituants inertes, un radical alcoxy comportant éventuellement des substituants inertes et contenant 1–4 atomes de carbone, un radical phénoxy ou un radical obtenu en éliminant les groupes chlorocarbonyle d'un chlorure d'acide carbamique organique n-fonctionnel contenant, au total, 2–10 atomes de carbone,
n représente un nombre entier de 1–3, et
z représente 0 ou 1.

2. Procédé selon la revendication 1, caractérisé en ce que, comme 5-acylimino-imidazolidinediones N,N'-disubstituées, on utilise celles de la formule générale mentionnée dans la revendication 1 dans laquelle
R et R' sont des radicaux identiques ou différents et représentent chacun un radical d'hydrocarbure aliphatique contenant 1 à 4 atomes de carbone,
R″ représente un radical d'hydrocarbure aliphatique n-valent, non substitué, saturé et contenant 1 à 6 atomes de carbone ou un radical d'hydrocarbure aromatique n-valent, non substitué et contenant 6 atomes de carbone,
n représente 1 ou 2, et
z représente 1.

3. Procédé selon les revendications 1 et 2, caractérisé en ce qu'on utilise les partenaires réactionnels en quantités correspondant à un rapport d'équivalents de 0,9:1 à 1,5:1 entre les groupes hydroxy alcooliques et les motifs structuraux d'imidazolidine-dione.

4. Utilisation des composés obtenus selon les revendications 1 à 3 et comportant, au total, au moins deux groupes hydroxy bloqués et libres comme matière de départ pour la fabrication de matières synthétiques de polyuréthanes.